# EUROPEAN PATENT SPECIFICATION

(11) **EP 1 118 664 B1**
(45) Date of publication and mention of the grant of the patent: **15.02.2006**
(21) Application number: 99970124.6
(22) Date of filing: 01.10.1999
(51) Int. Cl.: C12N 15/85, A01K 67/027, C12N 15/37, C12N 5/18, C12N 15/63

(54) **Method for producing choroid plexus immortalized cell lines**
Methode zur Herstellung von Choroid Plexus immortalisierten Zelllinien
Méthode de production de cellules de choroid plexus immortalisées

(30) Priority: 02.10.1998 JP 29613898; 02.10.1998 JP 29613998
(43) Date of publication of application: 25.07.2001
(73) Proprietor: Fact Co., Ltd., Sendai-shi Miyagi 989-3204 (JP)
(72) Inventor: HOSOYA, Kenichi, Sendai-shi, Miyagi 982-0826 (JP); TERASAKI, Tetsuya, Sendai-shi, Miyagi 981-3101 (JP); UEDA, Masatsugu, Kawagoe-shi, Saitama 350-1151 (JP); OBINATA, Masuo, Sendai-shi, Miyagi 980-0871 (JP)
(74) Representative: Forstmeyer, Dietmar
(86) International application number: PCT/JP1999/005423
(87) International publication number: WO 2000/020599

(56) References cited:
- EP-A- 0 942 066
- WO-A-97/39117
- JAT P ET AL: "DIRECT DERIVATION OF CONDITIONALLY IMMORTAL CELL LINES FROM AN H-2K-B-TSA58 TRANSGENIC MOUSE" PROCEEDINGS OF THE NATIONAL ACADEMY OF SCIENCES OF THE UNITED STATES, vol. 88, no. 12, 1991, pages 5096-5100, XP002221051 1991 ISSN: 0027-8424
- GREENWOOD J: "CHARACTERIZATION OF A RAT RETINAL ENDOTHELIAL CELL CULTURE AND THE EXPRESSION OF P GLYCOPROTEIN IN BRAIN AND RETINAL ENDOTHELIUM IN-VITRO" JOURNAL OF NEUROIMMUNOLOGY, vol. 39, no. 1-2, 1992, pages 123-132, XP009000939 ISSN: 0165-5728
- GREENWOOD J ET AL: "SV40 LARGE T IMMORTALISED CELL LINES OF THE RAT BLOOD-BRAIN AND BLOOD-RETINAL BARRIERS RETAIN THEIR PHENOTYPIC AND IMMUNOLOGICAL CHARACTERISTICS" JOURNAL OF NEUROIMMUNOLOGY, ELSEVIER SCIENCE PUBLISHERS BV, XX, vol. 71, 1996, pages 51-63, XP000926618 ISSN: 0165-5728
- OBINATA MASUO: "Conditionally immortalized cell lines with differentiated functions established from temperature-sensitive T-antigen transgenic mice." GENES TO CELLS, vol. 2, no. 4, 1997, pages 235-244, XP009000919 ISSN: 1356-9597
- NOBLE MARK: "Production and growth of conditionally immortal cell lines from the H-2KbtsA58 transgenic mouse." 1999 , METHODS IN MOLECULAR BIOLOGY, VOL. 97, PAGE(S) 139-158 , PROTOCOLS. 1999 HUMANA PRESS INC. SUITE 808, 999 RIVERVIEW DRIVE, TOTOWA, NEW JERSEY 07512, USA XP001120423 ISBN: 0-89603-387-2 * the whole document *
- NOBLE M. ET AL.: 'The H-2KbtsA58 transgenic mouse: a new tool for the rapid generation of novel cell lines' TRANSGENIC RESEARCH vol. 4, no. 4, 1995, pages 215 - 225, XP002935508
- KAZUHISA HAYAKAWA ET AL.: 'The cultured capillary endothelial cells derived from bovine retinas- the change in forms by adding glucose-' NEW OPHTHALMOLOGY vol. 5, no. 6, 1988, pages 931 - 936, XP002935509
- GILLIES M.C. ET AL.: 'Effect of high glucose on permeability of retinal capillary endothelium in vitro' INVESTIGATIVE OPHTHALMOLOGY & VISUAL SCIENCE vol. 38, no. 3, 1997, pages 635 - 642, XP002935510
- RAMANATHAN V.K. ET AL.: 'Primary cell culture of the rabbit choroid plexus: an experimental system to investigate membrane transport' PHARMACEUTICAL RESEARCH vol. 13, no. 6, 1996, pages 952 - 956, XP002935511
- HAKVOORT A. ET AL.: 'The polarity opf choroid plexus epithelial cells in vitro is improved in serum-free medium' JOURNAL OF NEUROCHEMISTRY vol. 71, no. 3, September 1998, pages 1141 - 1150, XP002935512
- TAKASHI TSURUO: 'Molecular structure and physiological system of the resistance of anticancer drug - Effects on blood-brain barrier' PHARMACOLOGY MAGAZINE vol. 115, no. 7, 1995, pages 513 - 522, XP002935513
- HOHEISEL D. ET AL.: 'Hydrocortisone reinforces the blood-brain barrier properties in a serum free cell culture system' BIOCHEMICAL AND BIOPHYSICAL RESEARCH COMMUNICATIONS 244 vol. 247, no. 2, March 1998, pages 312 - 316, XP002935514

## Description

### TECHNICAL FIELD

The present invention relates to immortalized cells established from a transgenic animal into which a large T-antigen gene of SV40 temperature sensitive mutant tsA58 has been introduced.

More specifically, the present invention relates to established cells derived from choroid plexus epithelial cells of the transgenic animal.

The established cells derived from choroid plexus epithelial cells of the present invention are useful for studying nutrition metabolism in the brain, studying permeation of drugs into the brain, and investigating the protection mechanism of metabolism and permeation of substances into the cerebrospinal system. These cells are therefore useful in screening drugs regarding the safety and efficacy thereof, and developing methods for diagnosing and treating diseases relating to nutrition metabolism disorders and homeostatic functional disorders of the brain on cellular level studies.

### BACKGROUND ART

Conventionally, tests for the assessment of safety and efficacy of drugs have been conducted using animals. However, to avoid use of a large number of animals from the viewpoint of animal right, technologies for in-vitro assessment of safety and efficacy of drugs using cultured cells are used on a practical level. For example, a technique of first testing using primary culture cells collected from living tissues or established immortalized cells which can infinitely proliferate, and then testing using animals is employed. The primary culture cells can initially proliferate very well, but the proliferation gradually declines as the subculture advances, and finally cells die out. This phenomenon is called cellular senescence. Furthermore, in addition to the fear that the characteristics of primary culture cells may differ each time they are collected from living tissues, the primary culture cells are said to change the characteristics as the subculture advances. Particularly, when the multiplication rate is very slow or when the cells are derived from a small organ, it is very difficult to obtain a sufficient amount of the primary culture cells for test. On the other hand, established culture cell which have acquired the capability of infinitely proliferating during subcultures of the primary culture cells can maintain stable characteristics. However, most of these cells no longer have part or all of the forms and functions possessed by the cells when they were in a living body. Therefore, it is difficult for such established cells to precisely reflect the original characteristics which the cell lines exhibited in the tissues from which they have been derived. In view of this situation, establishment of immortalized cells which can continuously maintain an active proliferation capability possessed by the primary culture cells without losing the characteristics inherently possessed by the cells during subculture, has been tried by transforming the cells by introducing oncogenes such as ras and c-myc, E1A gene of adenovirus, large T-antigen gene of SV40 virus, or HPV16 gene of human papillomavirus. Such immortalized cells which are derived, from some organs lose several functions at the time of introducing oncogenes or large T-antigen genes after preparation of a primary culture cell. Thus, acquisition of immortalized cells in the stringent meaning of holding an original function has been difficult. Preparing a primary culture cell and acquiring a cell line has been very difficult, particularly when the multiplication rate is very slow or when the cells are derived from a small organization.

To overcome these problems, a method of establishing immortalized cells by applying a recently developed transgenic technology to individual animals has been proposed. Instead of introducing oncogenes or large T-antigen genes into individual cells, according to this method, transgenic animals into which these genes have been introduced in chromosomes in a stable manner are prepared. Then, a primary culture cell is prepared from an organ of these animals which possesses the oncogenes or large T-antigen genes in the cells at the time of development of the individuals. The primary culture cells is subcultured to establish immortalized cells. In particular, immortalized cells are easily available from organs of transgenic mice into which a large T-antigen gene of a temperature sensitive mutant tsA58 of SV40 has been introduced. The immortalized cells are very useful because growth of the resulting cells and expression of the differentiation character can be managed by changing the temperature (Noble M. et al. (1995) Transgenic Research 4, 215-225; ObinataM. (1997) GenestoCells2, 235-244. Ratshaving a body weight about ten times that of mice are advantageous for preparing cells used for the establishment of a cell line from various organs, particularly for preparing a cell line originating from small organs such as retinal capillary endothelial cells or intracerebral cells (e.g. choroid plexus epithelial cells, capillary vessel endothelial cells, etc.), because primary culture cells or many other cells can be easily obtained by separating organs or tissues from the rats. Therefore, transgenic rats into which a large T-antigen gene of a temperature sensitive mutant tsA58 of SV40 has been introduced, which are useful for establishing immortalized cells due to easy availability from various organs and the capability of controlling the growth of the resulting cells and expression of the differentiation character by changing temperatures, had already been produced.

On the other hand, in research investigating the effect and mechanism of nerve drugs on the blood-cerebrospinal fluid barrier mechanism, a method of using a primary culture cell of choroid plexus epithelial cells in place of animal tests is being developed in view of animal right. In this instance, because it is difficult to constantly obtain a sufficient amount of culture cells for the test from small animals, effective cell lines usable in place of such culture cells have been strongly desired.

### DISCLOSURE OF THE INVENTION

In view of this situation, the present inventors have conducted extensive studies and, as a result, have established immortalized cells from transgenic rats intowhich immortalizing genes have been introduced by separating an epithelial cell line from the choroid plexus of brain.

Still another obj ect of the present invention is therefore to provide established cells derived from choroid plexus epithelial cells, capable of expressing a temperature sensitive SV40 large T-antigen gene, showing localization of Na⁺ -K⁺ ATPase and GLUT-1 transport carriers in the cell membrane, and when cultured in a monolayer, showing the localization of Na⁺ -K⁺ ATPase in the apical side.

A further object of the present invention is to provide a method of establishing such immortalized cells using a large T-antigen gene of the SV40 temperature sensitive mutant tsA58.

The present invention has been completed to achieve the above objects and relates to immortalized cells established from a transgenic animal into which a large T-antigen gene of SV40 temperature sensitive mutant tsA58 has been introduced. More specifically, the present invention relates to the established cells derived from choroid plexus epithelial cells of such a transgenic animal. Specifically, the present invention relates to established cells expressing a temperature sensitive SV40 large T-antigen gene, showing localization of Na⁺ -K⁺ ATPase and GLUT-1 transport carriers in the cell membrane, and when cultured in a monolayer, showing the localization of Na⁺ -K⁺ ATPase in the apical side. The cells deposited in National Institute of Bioscience and Human-Technology, Agency of Industrial Science and Technology, the Ministry of International Trade and Industries, under the deposition number FERM BP-6508 can be given as such established cells.

The present invention also relates to a method of establishing immortalized cells comprising treating the choroidal plexus tissues of such a transgenic animal with protease, selecting the cells exhibiting an epithelial cell-like/paving stone-like form from the resulting cells, and subculturing such cells. The rat can be given as an example of such a transgenic animal.

Furthermore, the present invention relates to the established cells obtained using such a method of establishment.

Due to the capability of forming tight junction among cells when cultured in a mono-layer on a porous flat membrane and the capability of reconstructing the blood-cerebrospinal fluid barrier with a inside-and-outside polarity in vitro, the established cells are useful for studying nutrition metabolism of the brain, studying permeation of drugs into the brain, and investigating the protection mechanism of metabolism and permeation of substances into the cerebrospinal system. These cells are therefore useful in screening drugs regarding the safety and efficacy thereof, and developing a method for diagnosing and treating diseases relating to nutrition metabolism disorders and homeostatic functional disorders of the brain on cellular level studies.

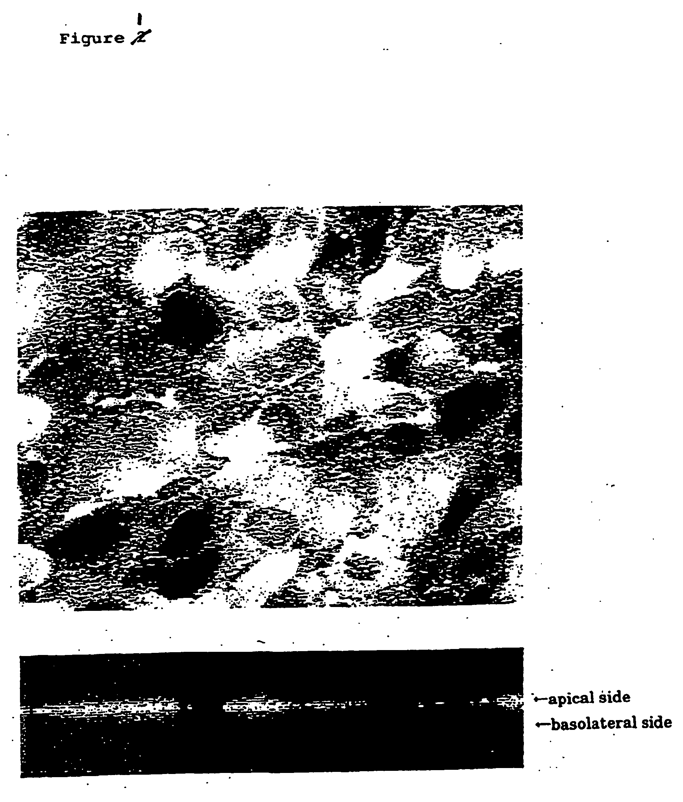

Figure 1 shows confocal laser scanning microscopy of Na⁺ -K⁺ ATPase of the established cell (TR-CSFB3) obtained in Example 10 of the present invention.

The upper photograph is a microscopic photograph of a plan view (XY section)of the cell wherein Na⁺ -K⁺ ATPase and GLUT-1 are seen to be expressed. The lower photograph is a microscopic photograph of a cross section view (XZ section)of the cell wherein Na⁺ -K⁺ ATPase are seen localized in apical side.

Figure 2 shows the proline active transport capability of the established cell (TR-CSFB3) obtained in Example 11 of the present invention.

Figure 3 shows interference of the proline active transport capability of the established cell (TR-CSFB3) obtained in Example 12 of the present invention by choline and ouabain.

### BEST MODE FOR CARRYING OUT THE INVENTION

The transgenic rat used in the present invention into which a large T-antigen gene of SV40 temperature sensitive mutant tsA58 has been introduced can be obtained as follows. Specifically, a whole genome DNA of tsA58ori(-)-2 which is produced from a large T-antigen gene of a temperature sensitive mutant tsA58 of SV40, for example, with deletion of the SV40 ori (replication origin), is linearized using a restriction endonuclease BamHI, and introduced into pBR322 to obtain a plasmid pSVtsA58ori(-)-2 (Ohno T. et al., Cytotechnology 7, 165-172 (1991)) pBR322. The plasmid is amplified in Escherichia coli in a large amount according to a conventional method.

The plasmid thus obtained is cut with a restriction endonuclease BamHI to eliminate a vector region. Because the DNA (5,240 bp) having a large T-antigen gene of tsA58 thus obtained has a promoter of the large T-antigen gene therein, a rat into which the DNA is introduced expresses this gene (the large T-antigen gene of tsA58) in all somatic cells.

Next, the resulting DNA is introduced into totipotent cells of rats in accordance with a conventional method to prepare transgenic rats having a temperature sensitive large T-antigen gene in all cells. As a totipotent cell, ES cells having totipotency can be given in addition to fertilized ova and early embryos. A microinjection method, electropolation method, liposome method, calcium phosphate method, and the like can be used for introducing DNA into ova and cultured cells.

Furthermore, the present gene can be introduced into ova by transplanting a nucleus of cultured cells into which a desired gene of the present invention has been introduced in enucleation unfertilized ova and initializing the ova (nuclear transplantation). However, as far as the efficiency of obtaining a transgenic rat is concerned, a transgenic rat having a large T-antigen gene of tsA58 incorporated into chromosomes of cells of each tissue at the time of development of individuals can be efficiently obtained by producing ova through microinjection of the gene of the present invention into male pronucleus of the pronucleus ova, transplanting the ova into the oviduct of an foster mother to obtain offspring, and selecting the offspring having the injected gene, thereby stably obtaining individuals into which the gene of the present invention has been incorporated.

Immortalized cells can be prepared with extracting cells (primary cells) from organs of gene-introduced rats thus obtained and repeating subculture of the cells. The resulting cells obtain the characters that the cells have the capability of permanently proliferating at 33-37°C and show the proper characteristics with terminating the proliferation at 39°C.

The brain of this rat is taken out to collect choroid plexus. The choroid plexus cut into pieces is treated with trypsin/EDTA to disperse cells. After terminating the enzymatic reaction by the addition of a culture medium containing fetal serum, the cells are collected by centrifugation and dispersed in a culture medium. The procedures of centrifugation and dispersion are repeated to wash the cells. The cells thus obtained are dispersed in a culture medium, inoculated on a culture plate, and incubated at 33°C. After subculturing three generations, colonies are formed. Colonies exhibiting a paving stone-like form inherent to epithelial cell and a comparatively fast growth rate are isolated from the surrounding cells using a penicillin cup. This procedure is repeated twice to isolate the cells originating from a single cell. The cells obtained are subjected to immunostaining to confirm localization of Na⁺ -K⁺ ATPase and GLUT-1 transporter on the cell membrane using a confocal laser scanning microscopy, whereby the cells are identified. The resulting cells exhibit a large T-antigen, maintain excellent proliferating activity after 50 generation subculture at 33°C, and express Na⁺ -K⁺ ATPase and GLUT-1 transporter. In particular, when the cells are cultured in a monolayer, Na⁺ -K⁺ ATPase which is present on the basolateral membrane side (a serous membrane) in other epithelial cells, is locally present in the apical side of the cell membrane.

### EXAMPLES

The present invention will now be described in more detail by way of examples, which are given for the purpose of explanation and should not be construed as limiting the present invention.

### Example 1

### Preparation of transgenic rat

A transgenic rat carrying DNA of an SV40 temperature sensitive mutant tsA58 was prepared according to the following method.

### (1) Preparation of a gene to be introduced

DNA of SV40 temperature sensitive mutant tsA58 was used for microinjection. The genome DNA of tsA58 was linearized using a restriction endonuclease BamHI and introduced into the BamH site of pBR322 to convert the Sfi I sequence to the SacII sequence, thereby obtaining a DNA clone pSVtsA58 ori (-)-2 with deletion of the SV40 ori site (replication origin) (See Ohno T. et al. , Cytotechnology 7, 165-172 (1991), Figure 1). The DNA was prepared from the pSVtsA58 ori (-) -2 according to a conventional method. Specifically, the pSVtsA58 ori(-)-2 of plasmid DNA obtained by amplification in Escherichia coli. was digested using a restriction endonucleases BamHI (made by Takara Shuzo Co., Ltd.) and the vector region was separated by agarose gel electrophoresis (1% gel; Boeringer company). Linear DNA fragment of tsA58 DNA with a length of 5240 bp were cut out from the gel. The gel was dissolved by agarase treatment (0.6 unit/100 mg gel: Agarase; Boeringer Co.). DNA was recovered by phenol-chloroform treatment and ethanol precipitation treatment. The purified DNA was dissolved in a TE buffer (10 mM Tris-HCl containing 1 mM EDTA, pH 7.6) to obtain a purified DNA solution with a concentration of 170 µg/mL. The DNA solution was diluted with a buffer (10 mM Tris-HCl containing 0.1 mM EDTA, pH 7.6) to a concentration of 5 µg/mL to obtain a DNA solution for microinjection. The resulting DNA solution was stored at -20°C until used for microinjection.

### (2) Preparation of transgenic rat

Microinjection of the DNA solution prepared in (1) above to the rat ova at pronucleus stage was carried out according to the following procedures. Sexually mature Wistar rats, aged eight weeks, were kept in a condition of a 12 hour light-and-shade cycle (light hours: 4:00-16:00) at 23±2°C and RH 55±5%. The estrous cycle of female rats was observed by vaginal smear to select the hormonal treating day. A pregnant-mare serum gonadotropic hormone (pregnant mare serum gonadotropin; PMSG, manufactured by Nippon Zenyaka Co.) was intraperitoneally administered at a dose of 150 IU/kg to female rats. After 48 hours, 75 IU/kg of human chorionic gonadotropin (hCG manufactured by Sankyo Zoki Co.) was administered thereby effecting superovulation treatment. The female and male rats were mated by being together in a cage. The ova at pronucleous stage were collected at 32 hours after the hCG administration by oviduct perfusion. AmKRB solution (Toyoda Y. and Chang M.C., J. Reprod. Fertil., 36, 9-22 (1974)) was used for the oviduct perfusion and incubation of ova. The collected (fertilized) ova were treated by an enzyme in an mKRB solution containing 0.1% hyaluronidase (Hyaluronidase TypeI-S, made by Sigma Co.) at 37°C for 5 minutes to remove cumulus cells. After washing three times with the mKRB solution to remove the enzyme, the fertilized ova were stored in a CO₂ incubator (5% CO₂-95% air, 37°C, saturated humidity) until DNA microinjection. A DNA solution was microinjected into the male pronucleus of the rat (fertilized) ova thus prepared. 228 ova after microinjection were transplanted in nine recipients (foster mothers) and 80 pups were obtained. The integration of the DNA was analyzed with DNA prepared from tails of the rats immediately after weaning by the PCR method (primers used: tsA58-1A, 5'-TCCTAATGTGCAGTCAGGTG-3' (corresponds to 1365-1384 sites), tsA58-1B, 5'-ATGACGAGCTTTGGCACTTG-3' (corresponds to 1571-1590 sites)). As a result, 20 rats (6 male, 8 female, and 6 unknown sexuality) were identified to have the gene introduced. Among these rats, 11 transgenic rat lines (male lines: #07-2, #07-5, #09-6, #12-3, #19-5, female lines: #09-7, #11-6, #12-5, #12-7, #18-5, #19-8) which survived as long as 12 weeks after elapse of the sexual maturation period were obtained. These G0 generation transgenic rats were mated with Wistar rats and established 2 lines of male founders (#07-2, #07-5) and 3 lines of female founders (#09-7, #11-6, #19-8), by confirming that the genes was introduced in germ line and transferred to next generation.

### Example 2

### Isolation of choroid plexus epithelial cells

In a clean bench, the brain was collected from one transgenic rat carring a large T-antigen gene of SV40 temperature sensitive mutant tsA58 obtained in Example 1. The choroid plexus from the inner wall of the right and left ventriculus lateralis through the upper wall of the third ventricle of the brain was collected and thoroughly washed with PBS. The tissue was cut into pieces with a volume of 1-2 mm³ in 2 mL of ice-cooled PBS. The tissue pieces were suspended into 1 mL of a 10X trypsin/EDTA solution (0.5% Trypsin, 0.53 mM EDTA; manufactured by Gibco BRL) to digest by the enzyme treatment at 37°C for 20 minutes. The tissue pieces were dispersed by gently stirring from time to time. The resulting cells were washed with a culture medium (DEME solution containing 10% FCS, 100 U/mL benzylpenicillin potassium, and 100 µg/mL streptomycin sulfate). The cells were dispersed in 2 mL of the culture and inoculated in a 35mm φ culture dish (Falcon, manufactured by Becton Dickinson Co.) and incubated (primary culture) at 33°C in a CO₂ incubator (5% CO₂-95% air, saturated humidity). Subculture was carried out at an interval of about one week using a trypsin/EDTA solution (0.05% Trypsin, 0.53 mM EDTA; manufactured by Gibco BRL) while replacing the medium twice a week. After subculture three times, 10²-10³ cells were inoculated in a 10 cm φ culture dish and incubated in a CO₂ incubator at 33°C to form colonies. After 7-10 days while replacing the medium twice a week, the colonies consisting of cells having a paving stone-like form inherent to epithelial cells which exhibit a comparatively fast growth rate were isolated from the surrounding cells using a penicillin cup. The cells which were obtained were again inoculated in a 10 cm φ culture dish and incubated at 33°C in a CO₂ incubator to form colonies. Colonies exhibiting a comparatively fast growth rate were isolated using a penicillin cup to obtain five lines of cells (TR-CSFB1, TR-CSFB2, TR-CSFB3, TR-CSFB4, TR-CSFB5).

TR-CSFB3 was deposited in National Institute of Bioscience and Human-Technology, Agency of Industrial Science and Technology, the Ministry of International Trade and Industries. The deposition number is FERM BP-6508.

### Example 3

### Confirmation of large T-antigen proteins

Expression of large T-antigen proteins in the five cell lines obtained in Example 2 were examined by the Western Blotting method (Experimental Medicine Separate Volume, Biotechnology Manual UP Series, "Cancer research protocol by the molecular biological approach", pages 108-115, YODOSHA Publishing Co., 1995) . The five cell lines (the 10^{th} generation) were cultured in a 90 mm φ culture dishes until saturation. The collected cells were solubilized using 1 mL of 3% SDS-PBS (pH 7.4) and unsolubilized fractions were removed by centrifugation at 10,000 rpm for 10 minutes, and then the total amount of proteins was determined by the Bradford method using the protein assay kit II of BIO-RAD Co. The proteins were separated by the SDS polyacrylamide gel electrophoresis in the amount of 20 µg each and transferred onto nitrocellulose membranes. The nitrocellulose membranes blocked by a 3% skimmed milk solution were reacted with an anti-SV40 large T-antigen mouse antibody (DP02-C, CALBIOCHEM Co.), as a primary antibody, and a HRP labeled anti-mouse IgG antibody A (Amersham Co.), as a secondary antibody, to detect the reactions specific to large T-antigen proteins using the ECL Western Blotting detection system (RPN2106M1 , a product of Amersham Co.). The results are shown in Table 1. As a result, the large T-antigen proteins were detected in all five cell lines.

**Table 1**

| Cells | TR-CSFB1 | TR-CSFB2 | TR-CSFB3 | TR-CSFB4 | TR-CSFB5 |
|---|---|---|---|---|---|
| T-Antigen | + | + | + | + | + |

### Example 4

### Confirmation of transport carrier of Na⁺ -K⁺ ATPase and GLUT-1

The cells obtained were cultured in a mono-layer and expression of Na⁺ -K⁺ ATPase and GLUT-1 transporter on the cell membrane was confirmed by confocal laser scanning microscopy observation of immunologically stained cells. the TR-CSFB3 cells obtained in Example 2 were cultured on a collagen coated cover glass in a 35 mm φ dish (a product of Falcon). Afterremoval of the culture solution, the cells were thoroughly washed with PBS, then 4 mL of a fixative (PBS containing 3% paraformaldehyde and 2% sucrose) was added. After allowing to stand at room temperature for 15 minutes, the cells were thoroughly washed with PBS. 2 mL of a blocking solution (Block Ace, manufactured by Dainippon Pharmaceutical Co. , Ltd.) was added and the mixture was allowed to stand for one hour at 37°C to effect blocking, followed by the reaction with a primary antibody (anti Na⁺ -K⁺ ATPase β2 rabbit antibody, a product of UBI, or anti-GLUT-1 rabbit antibody, a product of Chemicon) for one hour at room temperature. The resulting product was washed four times with PBS and reacted with a secondary antibody (FITC labeled anti-rabbit IgG, a product of Capel) for one hour at room temperature, followed by washing with PBS four times. Finally, labeled cells were sealed with a glycerol sealing solution (a 90% glycerol solution in PBS containing 0.1% (v/v) of Perma Fluor (a product of Lipshaw)). The cover glass periphery was sealed with a manicure. A confocal laser scanning microscopy (CLSM; Zwiss LSM 410, manufactured by Zwiss) was used for the observation. As a result, as shown in Figure 1, expression of Na⁺ -K⁺ ATPase and GLUT-1 transporter were detected in TR-CSFB3 cells. In particular, Na⁺ -K⁺ ATPase which is present on the basolateral membrane side (a serous membrane side) in other epithelial cells was seen to be locally present in the apical side of the cell membrane, confirming that the cells are choroid plexus epithelial cells. The same results were obtained with other cells.

### Example 5

### Confirmation of proline transport capability

The concentration dependency of the resulting cells on the L-proline transport was examined to determine the L-proline transport capability. This was compared with the reported values of L-proline transport capability in the choroid plexus, thereby confirming that the resulting cells have functions as the choroid plexus epithelial cells.

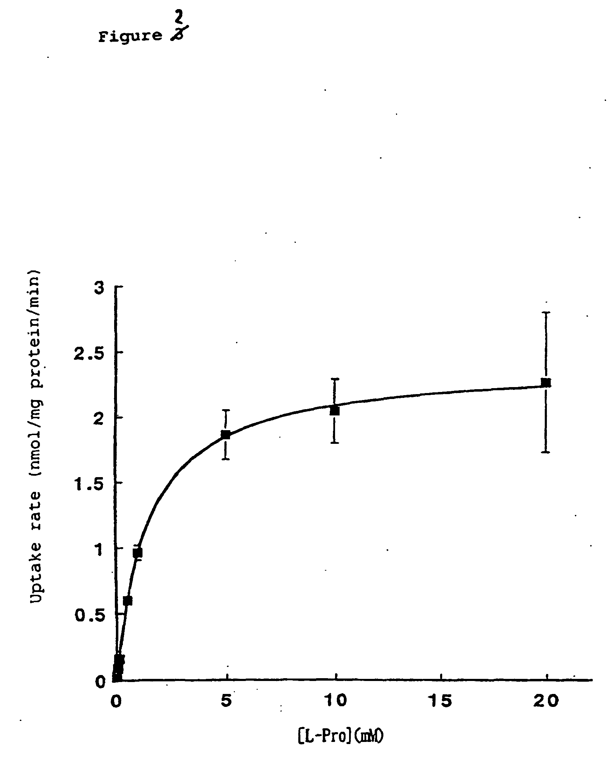

Specifically, TR-CSFB3 cells obatined in Example 2 were inoculated in a 24-well cell culture plate at a concentration of 3x10⁵ cells/well/mL and incubated for 24 hours at 33°C in a CO₂ incubator to be the cells confluent. After removal of the medium by aspiration, the cells were washed with a previously heated (37°C) uptake buffer (1), which was prepared from a solution which contains 122 mM NaCl, 3 mM KCl, 1.4 mM CaCl₂, 1.4 mM MgSO₄ •7H₂O, 0.4 mM K₂HPO₄, 10 mM Hepes, and 25 mM NaHCO₃ by bubbling 5% CO₂-95% O₂ into the solution for 20 minutes and adjusting the pH of the resulting solution to 7.4 with NaOH. 0.2 mL of uptakebuffer (1) containing 185KBq/mL of [³H]-L-proline and heated to 37°C was added. Solutions containing proline at different concentrations were prepared by adding non-labeled L-proline to uptake buffer (1) to make final concentrations of 0.005, 0.01, 0.05, 0.1, 0.5, 1, 5, 10, 20 mM. After the uptake reaction for 30 minutes and washing three times with PBS, 1 mL of PBS containing 1% Triton X-100 was added and the mixture was allowed to stand overnight to solubilize the cells. The radioactivity was measured using a liquid scintillation counter (LS-6500 made by Beckmann Co.). In addition, the amount of proteins was determined using a protein assay kit manufactured by Bio-Rad Co. Using the plot formula for the uptake rate vs. the L-proline concentration (V = Vmax X[S] / (Km + [S]), wherein Vmax indicates a maximum velocity constant, Km indicates the Michaelis constant, and [s] is a substrate concentration), the Km and the Vmax for L-proline uptake were analyzed using the non-linear minimum square program (Yamaoka K. et al. (1981) J. Pharmacobio-Dyn., 4, 879-885). The results are shown in Figure 2. As a result, it was confirmed that the uptake of L-proline ([³H]-L-proline) was concentration-dependent, the Km was 1.5 mM, and the Vmax was 2.4 nmol/min/mg protein. The value for Km as determined was similar to the Km value from rabbit choroid plexus of 1.1 mM (CobenL.A. et al. (1972) Brain Res., 30, 67-82) . This confirms that the resulting cells possess the function of choroid plexus epithelial cell line.

### Example 6

### Inhibition of proline active transport by choline and ouabain

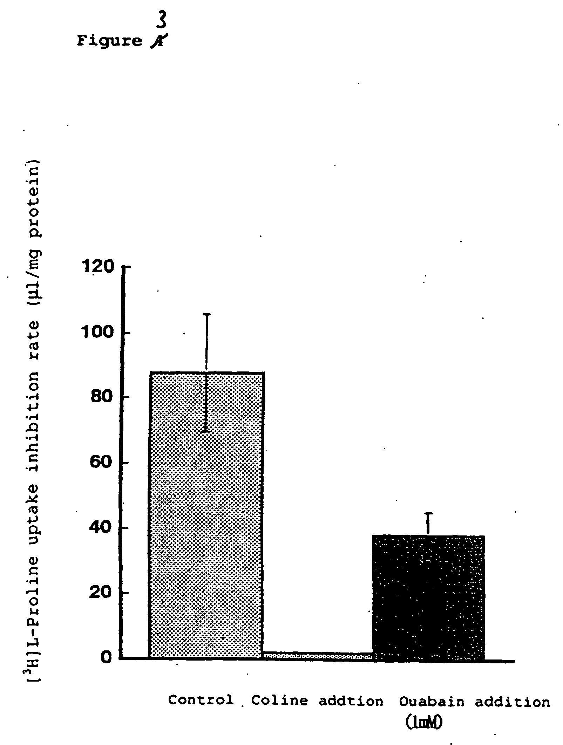

The L-proline uptake into the isolated choroid plexus is dependent on Na⁺. Therefore, the Na⁺ dependency of the L-proline uptake by the cells obtained was confirmed, and then the cells were confirmed to have functions as the choroid plexus epithelial cells in the same way as in Example 5. However, because the experiment must be carried out under Na⁺-free conditions, all Na⁺ in the uptake buffer (1) was replaced with coline. For the confirmation of the effect of ouabain, the uptake buffer (1) containing a tracer to which 1 mM of ouabain was added was used (because ouabain is an inhibitor of Na⁺ -K⁺ ATPase, the concentration gradient of Na⁺ is disappeared.). Both reactions were carried out for 30 minutes. The results are shown in Figure 3. It was confirmed that L-proline uptake was inhibited as much as 98% under Na⁺-free conditions. It was confirmed that 56% of L-proline uptake was inhibited by 1 mM ouabain. As a result, the L-proline uptake of TR-CSFB3 cells was confirmed to be Na⁺-dependent. This confirms that the resulting cells possess the function of choroid plexus epithelial cell line.

Cell lines derived from choroid plexus epithelial cells are provided. The cells express a temperature sensitive SV40 large T-antigen gene, show localization of Na⁺ -K⁺ ATPase and GLUT-1 transporter in the cell membrane, and when cultured in a monolayer, show the localization of Na⁺ -K⁺ ATPase in the apical side. Also provided is a method of establishing immortalized cells, which derived from choroidal tissues of a transgenic animal carrying a large T-antigen gene of an SV40 temperature sensitive mutant tsA58 by protease treatment.

Due to the capability of forming tight junctions among cells when cultured in a mono-layer on a porous flat membrane and the capability of reconstructing the blood-cerebrospinal fluid barrier with a inside-and-outside polarity in vitro, the established cells are useful for studying nutrition metabolism in the brain, studying permeation of drugs into the brain, and investigating the protection mechanism of metabolism and permeation of substances into the cerebrospinal system. These cells are therefore useful in screening drugs regarding the safety and efficacy thereof, and developing a method for diagnosing and treating diseases relating to nutrition metabolism disorders and homeostatic functional disorders of the brain in cellular level studies.

### REMARKS TO DEPOSITED MICROORGANISM

Name and address of the organization in which the microorganism has been deposited:
Name: National Institute of Bioscience and Human-Technology, Agency of Industrial Science and Technology, The Ministry of International Trade and Industry
Address: 1-1-3, Higashi, Tsukuba-shi, Ibaraki-ken, Japan (Postal Code: 305-3566).
Date of deposition: September 18, 1998
Number of deposition given by the deposition organization: FERM BP-6508

## Claims

1. A method to establish an immortalized choroid plexus epithelial cell which expresses a temperature sensitive SV40 large T-antigen gene, wherein the method comprises the following steps:
a) microinjecting a large T-antigen gene of SV40 temperature sensitive mutant tsA58 to a male pronucleus of the pronucleus ova of a rat, and transplanting the ova into the oviduct of a foster-mother to obtain a transgenic rat,
b) treating choroid plexus epithelial cells of said transgenic rat with protease, and
c) subculturing the resulting cells,
**characterized in that** the established cell expresses a temperature sensitive SV40 large T-antigen gene, shows localization of Na⁺-K⁺ ATPase and GLUT-1 transporter in the cell membrane, and when cultured in a monolager, shows the localization of Na⁺-K⁺ ATPase in the opical side and preferentially is FERM BP-6508.

## Patentansprüche

1. Verfahren zum Etablieren immortalisierter Epithelzellen eines Choroid-Plexus, welche ein temperaturempfindliches großes T-Antigen-Gen von SV40 exprimieren, wobei das Verfahren die folgenden Schritte umfasst:
a) Mikroinjektion des großen T-Antigen-Gens der temperaturempfindlichen Mutante tsA58 von SV40 in den männlichen Pronucleus des Pronucleus-Eies einer Ratte, und Transplantieren des Eies in den Eileiter einer Leihmutter, um eine transgene Ratte zu erhalten;
b) Behandeln der Epithelzellen des Choroid-Plexus der transgenen Ratte mit Protease, und
c) Subkultivieren der erhaltenen Zellen,
**dadurch gekennzeichnet, dass** die etablierte Zelle ein temperaturempfindliches, großes T-Antigen-Gen von SV40 exprimiert, eine Lokalisation der Na⁺-K⁺-ATPase und des GLUT-I-Transporters in der Zellmembran zeigt, und, wenn sie in einer Monoschicht kultiviert wird, die Lokalisation der Na⁺-K⁺-ATPase an der apikalen Stelle zeigt und vorzugsweise FERM-BP-6508 ist.

## Revendications

1. Méthode pour établir une cellule épithéliale de choroïde plexus immortalisée qui exprime un gène du gros antigène T sensible à la température de SV40, dans laquelle la méthode comprend les étapes suivantes :
a) micro-injection d'un gène du gros antigène T d'un mutant tsA58 sensible à la température de SV40 à un pronucleus mâle des ovules de pronucleus d'un rat, et transplantation des ovules dans l'oviducte d'une mère porteuse pour obtenir un rat transgénique,
b) traitement des cellules épithéliales de choroïde plexus dudit rat transgénique avec une protéase, et
c) mise en sous-culture des cellules résultantes,
**caractérisée en ce que** la cellule établie exprime un gène du gros antigène T sensible à la température de SV40, présente une localisation du transporteur de Na⁺-K⁺ ATPase et GLUT-1 dans la membrane cellulaire, et lorsqu'elle est mise en culture dans une couche monocellulaire, présente la localisation de Na⁺-K⁺ ATPase dans la face apicale et est de préférence FERM BP-6508.
